# EUROPEAN PATENT SPECIFICATION

(11) **EP 3 993 845 B1**
(45) Date of publication and mention of the grant of the patent: **14.10.2026**
(21) Application number: 20733468.1
(22) Date of filing: 23.06.2020
(51) Int. Cl.: A61L 29/08

(54) **FUNCTIONALIZED BALLOON SURFACE**
FUNKTIONALISIERTE BALLONOBERFLÄCHE
SURFACE DU BALLONNET FONCTIONNALISÉE

(30) Priority: 02.07.2019 EP 19183858
(43) Date of publication of application: 11.05.2022
(73) Proprietor: BIOTRONIK AG, 8180 Bülach (CH)
(72) Inventor: QUINT, Bodo, 79802 Dettighofen (DE); WERNLI, Jeremy, 5430 Wettingen (CH)
(74) Representative: WSL Patentanwälte Partnerschaft mbB
(86) International application number: PCT/EP2020/067463
(87) International publication number: WO 2021/001209

(56) References cited:
- EP-A2- 2 248 541
- WO-A1-2017/079328
- WO-A2-2014/125465
- US-A1- 2008 157 444
- US-A1- 2013 090 599
- DATABASE WPI Week 200419, Derwent World Patents Index; AN 2004-194319, XP002796257
- DATABASE WPI Week 201816, Derwent World Patents Index; AN 2018-13053P, XP002796258

## Description

Surfaces of balloons used, for example, in balloon catheter systems, for example for implanting stents, are particularly important since an appropriate modification allows the stent retention force on the balloon to be increased, for example, or the properties of the balloon surface as a carrier for additional components, such as electrical lines or drug coatings to be improved.

Known drug-coated balloons are often associated with the problem that, for example, coagulate coatings used in the process are comparatively thick. Further, it is difficult that carrier membranes are often too finely structured and, as a result, are not able to sufficiently receive an existing coating formulation, so that the structure-related adhesion promotion does not occur in such a case. In this regard, it is disclosed in WO 2014/125465 A2 a method for loading structures surfaces. In JP2003225301A a method of coating a medical appliance and in KR20180012885A a method of coating a balloon catheter is disclosed. Further, in US 2008/0157444 A1 a method of making a fiber-reinforced medical balloon and in EP 2248541 A2 a method for producing a balloon catheter is disclosed. Also, in WO 2017/079328 A1 a method of making an electrospun fiber is shown. US 2013/090599 A1 discloses a balloon catheter with measuring electrodes.

During the stent expansion, intensive shearing forces arise in stent delivery systems, wherein the adhesion of the coating to the balloon itself, or the strength of the coating, are often limiting. There is a risk due to abrasion of the coating or the delamination thereof (particle emissions).

Moreover, printing inks and electrically printable conductive tracks regularly use mild, and preferably safe, solvent systems, which are less suitable for polymer systems and can themselves be very easily dissolved by common solvents, such as alcohols or acetone. For this reason, some of these technical systems are additionally refined with a chemical curing mechanism, which, in general, is triggered thermally. However, since conventional printed circuit board materials are considerably more stable thermally than balloon surfaces, the process conditions required here are difficult to implement. With insufficient thermal fixation, these printed structures are quickly partially dissolved by subsequent application layers.

Proceeding from this, it is the object of the present invention to provide a method for producing a balloon for a balloon catheter, so that a balloon surface is created which provides increased friction, or improved adhesion promotion to subsequent coatings or a crimped stent, or protection for a further component of the balloon.

This object is achieved by a method having the features of claim 1, by a balloon having the features of claim 8, and by a use having the features of claim 10. Advantageous embodiments of these aspects of the invention are provided in the corresponding dependent claims and are described hereafter.

Accordingly, a method for producing a balloon, in particular for a balloon catheter, is disclosed, comprising the following steps:
- providing the balloon, wherein the balloon has an outer surface;
- providing a solution, comprising a solvent and a polymer dissolved therein; and
- depositing the polymer onto the surface for forming a surface coating, comprising the polymer, on the surface of the balloon.

The method according to the invention allows a surface coating to be produced, which has a roughened surface composed, for example, of a polymer-analogous material of the balloon material. This material is, in particular, inherently joined to a base material of the balloon by way of a swelling/partial solution process. The created surface coating, in general, includes a "nonwoven-like" structuring since predominantly fiber-like particle structures are created, even under spray conditions due to the spraying action and minimal surface tension of the solvent used. In particular, it is possible to achieve coating wall thicknesses of less than 5 micrometers.

According to one aspect of the invention, the method uses as the solvent: trifluoroacetic acid (C₂HF₃O₂, CAS number 76-05-1).

According to one embodiment of the method, it is further provided that the polymer (11) is one of the following polymers: an aliphatic or aromatic polyamide, or a thermoplastic polymer such as a polyether block amid copolymer, for example PEBAX 6233.

According to one embodiment of the method, it is further provided that the polymer, in the solution, has a concentration in the range of 1 wt.% to 30 wt.%, and preferably 1 to 10 wt.%. In one embodiment, it is preferred when the polymer, in the solution, has a concentration in the range of 15 to 25 wt.%. In particular, an electrospinning process can advantageously be carried out in such a range. In another embodiment, it is preferred when the polymer, in the solution, has a concentration in the range of 1 to 20 wt.%. The electrospinning process can be carried out particularly advantageously in such an embodiment.

According to one embodiment of the method, it is further provided that, for the deposition of the polymer on the surface of the balloon, the solution is sprayed by way of a nozzle in a direction toward the surface, wherein, in particular, fibrous structures of the polymer are formed. This can be achieved, for example, by an airbrush method or an electrospinning process.

According to one embodiment of the method, it is further provided that the nozzle has an inside diameter in the range of 0.1 mm to 1.2 mm.

According to one embodiment of the method, it is further provided that the nozzle has a distance from the surface of the balloon of greater than or equal to 10 cm during spraying of the solution.

According to a further preferred embodiment of the method, it is provided that polymer of the solution is deposited or applied onto the surface of the balloon in fibrous form.

According to one embodiment of the method, it is further provided that the balloon comprises one of the following substances or is formed of one of the following substances: an aliphatic or aromatic polyamide or a thermoplastic elastomer-modified polymer, such as a polyether block amide (PEBA).

According to one embodiment of the method, it is further provided that the surface coating is coated with a drug or a composition containing a drug.

According to another aspect of the method, it is further provided that the balloon comprises at least one electrical conductor on the surface of the balloon prior to the deposition of the polymer, and wherein the generated surface coating preferably protrudes beyond a region of the surface coating covering the at least one conductor, in a manner normal to the surface of the balloon in a surrounding area of the at least one conductor. The at least one conductor can be created, for example, by printing the surface of the balloon using an electrically conductive ink.

Another aspect of the present invention relates to a balloon for a balloon catheter, wherein the balloon is produced by way of the methods according to the invention.

Another aspect of the present invention relates to a balloon catheter, comprising a balloon according to the invention and a stent arranged on the surface coating of the balloon.

Another aspect of the present invention relates to a use of a solution, comprising trifluoroacetic acid, as the solvent, and a polymer dissolved in the solvent for coating an outer surface of a balloon.

The polymer can have the above-described concentration in the solution. The polymer can further be one of the polymers described above in the present connection.

Embodiments and further features and advantages of the invention will be described hereafter based on the figures. In the drawings:
- FIG. 1: shows a schematic illustration of one embodiment of the method according to the invention;
- FIG. 2: shows a photographic representation (200x magnification) of a rough, homogeneously distributed surface coating made of a polyether block amide Pebax 6333, which was created by way of the method according to the invention;
- FIG. 3: shows a photographic representation (200x magnification) of a surrounding area of a printed conductor structure on a balloon generated by way of the method according to the invention; and
- FIG. 4: shows a photographic representation (50x magnification) of a surface coating generated by way of the method according to the invention.

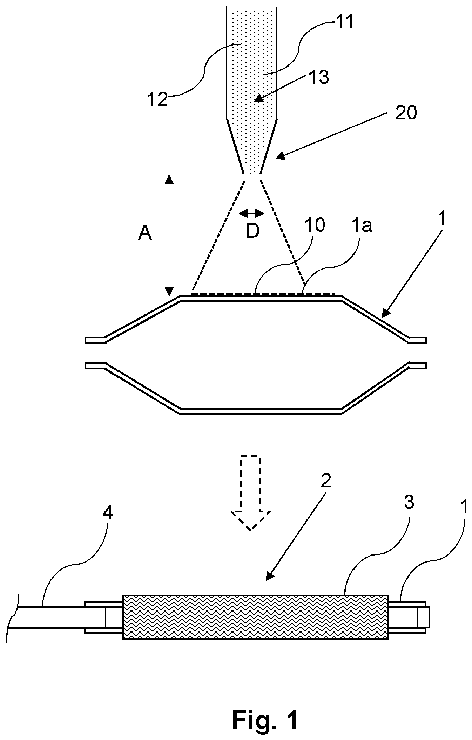

FIG. 1 shows a schematic illustration of one aspect of a method according to the invention for producing a balloon 1 for a balloon catheter 2, comprising the following steps:
- providing the balloon 1, wherein the balloon 1 has an outer surface 1a;
- providing a solution 13, comprising a solvent 11 and a polymer 12 dissolved therein, wherein Pebax 6233 is dissolved here, for example, as the polymer 12 in trifluoroacetic acid in a concentration of 1 wt.% to 5 wt.%; and
- depositing the polymer 12 onto the surface 1a for forming a surface coating 10, comprising the polymer 12, on the surface 1a of the balloon 1.

In this regard, the trifluoroacetic acid advantageously has a very unusual wetting behavior since this compound is based on a very small molecule that is "ultrahydrophobic" at one end and, at the opposite molecule end, represents a "highly polar" organic acid. Due to this structure, it tends to virtually crawl out of glass/porcelain vessels, for example. Despite the acidic effect and the high polarity, this compound forms surfaces having very low surface tension and very good wetting action.

A plurality of polyamides, as well as the PEBA copolymers typical in catheter production, are soluble in trifluoroacetic acid. Spraying surfaces 1a of such balloons with the aforementioned solution 13 leads to surprising results.

For example, at a spraying distance A between the nozzle 20 and the surface 1a of greater than or equal to 10 cm, and an inside diameter D of the nozzle 20 in the range of 0.1 mm to 1.2 mm, as well as a spraying pressure of approximately 3 bar, it was possible to observe gel-like depositions on the surface 1a, which still cause relaxations at the balloon 1, but no longer deform the balloon to any great degree. Despite spraying on one side, straight components without notable deformation were obtained with a support pressure of the balloon made of PA12 of 1 bar. It was surprising, however, that surface depositions that were still rough and have a nonwoven-like structure obtained across a very large dilution range.

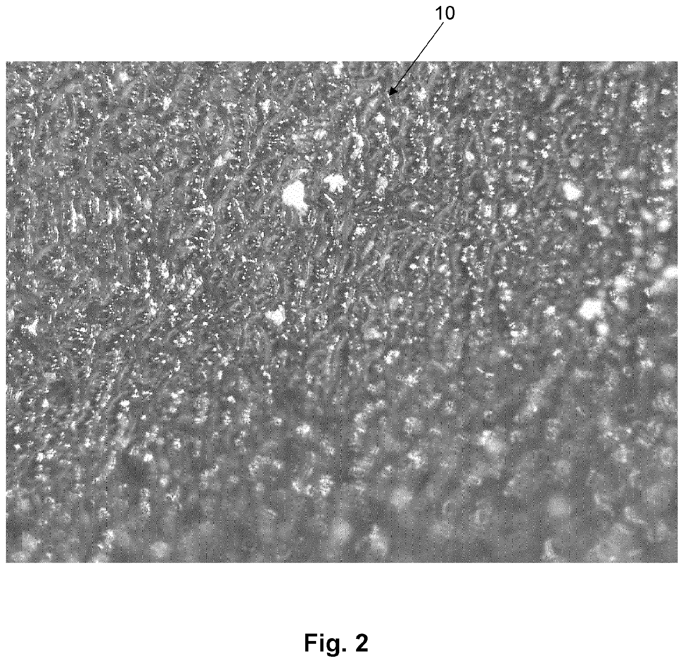

This structure is identified in FIGS. 2 to 4 by dyeing of the coating solution 13 with rhodamine. At >> 100-fold magnifications, a complex surface structure that is structured in the micrometer range is apparent (see FIG. 2).

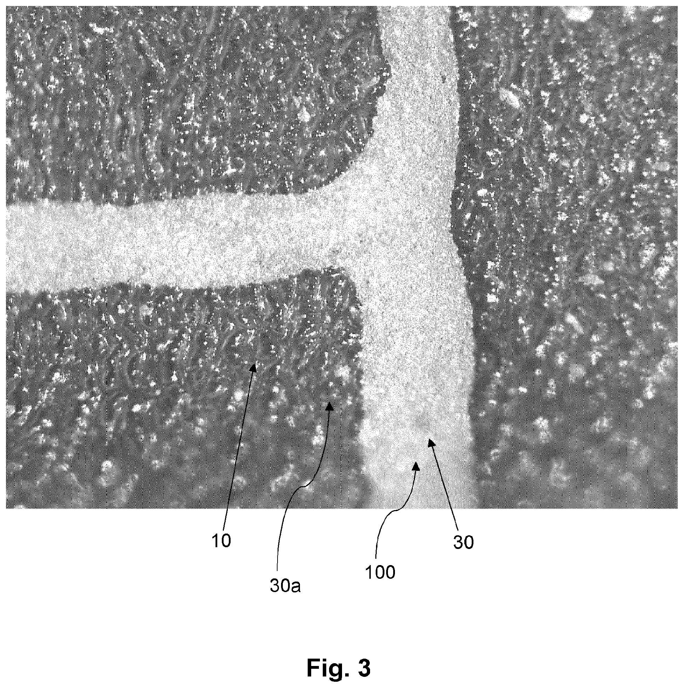

The balloon 1 to be coated can further comprise a printed conductor structure, for example, such as in the form of an electrical conductor 30. FIG. 3 shows in this regard that the surface coating 10 on the balloon 1 in the surrounding area of the conductors 30 spontaneously gels as a result of a loss of solvent to the underlying polyamide, while the wetting effect outweighs on the particular conductor 30, resulting in a planar and thin insulating coating 100 on this substrate. The respective conductor 30 is thus mechanically embedded into the newly created surface by way of a high-strength, flexible material, and can thus be protected against peeling or shear load.

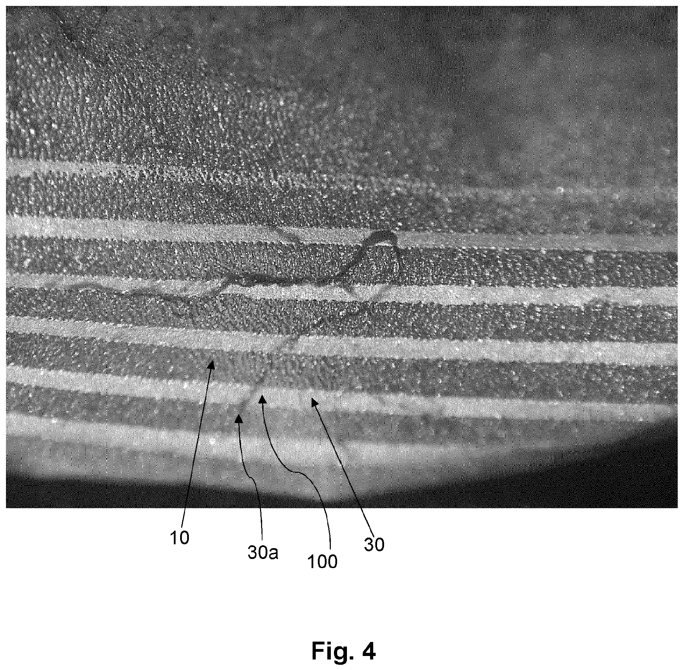

According to FIG. 4 (50x magnification), the surrounding spray region located further away also includes spider web-like deposits when the spray coating is implemented using a needle-shaped nozzle arrangement.

Due to the low surface tension of the trifluoroacetic acid and the viscosity of the polymer solutions, the spraying process (and the utilized nozzle geometry) preferably creates thread-shaped fragments, which are preserved in the gas phase due to solvent loss.

As a result of the low surface tension, the polymer 12 is pulled off in a thread-like manner in the pointed nozzle 20, and this structure will be preserved approximately unchanged during the spray phase due to the high vapor pressure. These fibrous particles are deposited on the surface 1a in gel-like form, where they suffer a considerable loss of volume due to the drying process, which is intensified by the suctioning or solvent-withdrawing action of the polyamide balloon surface 1a.

Since trifluoroacetic acid represents an intensive solvent for polyamide 12, this residual moisture creates solvent adhesion to the substrate. This surface cannot be abraded/separated from the surface 1a even with intensive abrasive action.

These properties, the low surface tension and very spontaneous gel formation predestine the solution 13 for an electrospinning or electrospraying process. Since both smaller material transfer volumes and greater control over the deposition can be achieved here, the electrospinning process is one of the preferred methods for applying the surface coating 10.

As is further shown in FIG. 1, the created surface structure is, in particular, also suitable for increasing the achievable stent retention force or for increasing adhesion of a drug coating.

In other steps of the method, the balloon 1 can be accordingly completed to form a catheter 2. The catheter 2 receives a shaft 4 in the process, wherein the balloon 1 is fixed at the distal end of the shaft 4, and wherein a stent 3 can be crimped onto the folded balloon 1 or onto the surface coating 10. Instead of a stent 3, the surface coating 10 of the balloon 1 can be provided with a drug coating.

The surface coatings 10 that can be produced according to the invention advantageously make it possible to introduce an applied active ingredient layer into the newly created surface structure, and thus allow large amounts of an active ingredient to be applied into the irregularities of the balloon coating,

The gain in friction with respect to the stent surface can contribute to an improved stent retention force in the case of balloon catheters 2.

The electrical insulation effect is ultimately limited by the surface roughness and structure of the created coating, but it is nonetheless apparent (see FIGS. 3 and 4) that thin coatings that are continuous across a surface area can be achieved after deposition. It is further advantageous that the solvent system 13 does not interact with the typical acrylate-based binders, whereby it is made possible to apply commercially available electrically conducting inks onto the particular balloon surface, without intensive thermal curing and disadvantageous delamination effects. One special feature in the process is the achievable mechanical protection of these printed structures 30 since the surrounding surface coating 10 gains in thickness more quickly than on the printed structures 30 themselves.

As a result of the roughly structured composition 10 adjoining the conductive tracks 30, an increased thickness compared to the particular conductive track 30 is achieved. This means that originally raised conducting electrical structures 30 are present in a mechanically protected and embedded manner after the coating has been carried out.

## Claims

1. A method for producing a balloon (1) for a balloon catheter (2), comprising the following steps:
- providing the balloon (1), wherein the balloon (1) has an outer surface (1a);
- providing a solution (13), comprising a solvent (11) and a polymer (12) dissolved therein; wherein the solvent (11) is trifluoroacetic acid, and
- depositing the polymer (12) onto the surface (1a) for forming a surface coating (10), comprising the polymer (12), on the surface (1a) of the balloon (1).

2. A method according to claim 1, wherein the polymer (12) is one of the following polymers: an aliphatic or aromatic polyamide, or a thermoplastic polymer, preferably a thermoplastic elastomer-modified polymer, and preferably a polyether block amide.

3. A method according to any one of the preceding claims, wherein the polymer (12), in the solution (13), has a concentration in the range of 1 wt.% to 30 wt.%.

4. A method according to any one of the preceding claims, wherein the polymer (12) of the solution (13) is deposited onto the surface (1a) by way of electrospinning.

5. A method according to any one of the preceding claims, wherein the balloon (1) comprises one of the following substances or is formed of one of the following substances: an aliphatic or aromatic polyamide or a thermoplastic elastomer-modified polymer, such as a polyether block amide.

6. A method according to any one of the preceding claims, wherein the surface coating (10) is coated with a drug or a composition containing a drug.

7. A method according to any one of the preceding claims, wherein the balloon (1) comprises at least one electrical conductor (30) on the surface (1a) of the balloon prior to the deposition of the polymer (12), and, in particular, the generated surface coating (10) protrudes beyond a region (100) of the surface coating (10) covering the at least one conductor (30), in a manner normal to the surface (1a) of the balloon (1) in a surrounding area (30a) of the at least one conductor (30).

8. A balloon (1) for a balloon catheter (2), wherein the balloon (1) is produced by way of the method according to any one of the preceding claims.

9. A balloon catheter (2), comprising a balloon (1) according to claim 8 and a stent (3) arranged on the surface coating (10) of the balloon (1).

10. Use of a solution (13), comprising trifluoroacetic acid, as the solvent (11), and a polymer (12) dissolved in the solvent (11) for coating an outer surface (1a) of a balloon (1).

## Patentansprüche

1. Verfahren zum Herstellen eines Ballons (1) für einen Ballonkatheter (2), umfassend die folgenden Schritte:
- Bereitstellen des Ballons (1), wobei der Ballon (1) eine Außenoberfläche (1a) aufweist;
- Bereitstellen einer Lösung (13), umfassend ein Lösungsmittel (11) und ein darin gelöstes Polymer (12); wobei das Lösungsmittel (11) Trifluoressigsäure ist, und
- Abscheiden des Polymers (12) auf die Oberfläche (1a) zum Ausbilden einer Oberflächenbeschichtung (10), umfassend das Polymer (12), auf der Oberfläche (1a) des Ballons (1).

2. Verfahren nach Anspruch 1, wobei das Polymer (12) eines der folgenden Polymere ist: ein aliphatisches oder aromatisches Polyamid oder ein thermoplastisches Polymer, vorzugsweise ein thermoplastisches elastomermodifiziertes Polymer und vorzugsweise ein Polyetherblockamid.

3. Verfahren nach einem der vorhergehenden Ansprüche, wobei das Polymer (12) in der Lösung (13) eine Konzentration im Bereich von 1 Gew.-% bis 30 Gew.-% aufweist.

4. Verfahren nach einem der vorhergehenden Ansprüche, wobei das Polymer (12) der Lösung (13) mittels Elektrospinnen auf die Oberfläche (1a) abgeschieden wird.

5. Verfahren nach einem der vorhergehenden Ansprüche, wobei der Ballon (1) einen der folgenden Stoffe umfasst oder aus einem der folgenden Stoffe gebildet ist: ein aliphatisches oder aromatisches Polyamid oder ein thermoplastisches elastomermodifiziertes Polymer, wie ein Polyetherblockamid.

6. Verfahren nach einem der vorhergehenden Ansprüche, wobei die Oberflächenbeschichtung (10) mit einem Wirkstoff oder einer Zusammensetzung, die einen Wirkstoff enthält, beschichtet wird.

7. Verfahren nach einem der vorhergehenden Ansprüche, wobei der Ballon (1) vor der Abscheidung des Polymers (12) mindestens einen elektrischen Leiter (30) auf der Oberfläche (1a) des Ballons umfasst und insbesondere die erzeugte Oberflächenbeschichtung (10) in einem Umgebungsbereich (30a) des mindestens einen Leiters (30) in einer Richtung normal zu der Oberfläche (1a) des Ballons (1) über einen Bereich (100) der Oberflächenbeschichtung (10), der den mindestens einen Leiter (30) bedeckt, hinausragt.

8. Ballon (1) für einen Ballonkatheter (2), wobei der Ballon (1) mittels des Verfahrens nach einem der vorhergehenden Ansprüche hergestellt ist.

9. Ballonkatheter (2), umfassend einen Ballon (1) nach Anspruch 8 und einen auf der Oberflächenbeschichtung (10) des Ballons (1) angeordneten Stent (3).

10. Verwendung einer Lösung (13), umfassend Trifluoressigsäure als das Lösungsmittel (11) und ein in dem Lösungsmittel (11) gelöstes Polymer (12), zum Beschichten einer Außenoberfläche (1a) eines Ballons (1).

## Revendications

1. Procédé de fabrication d'un ballonnet (1) pour un cathéter à ballonnet (2), comprenant les étapes suivantes:
- fournir le ballonnet (1), le ballonnet (1) présentant une surface extérieure (1a);
- fournir une solution (13), comprenant un solvant (11) et un polymère (12) dissous dans celui-ci; dans lequel le solvant (11) est l'acide trifluoroacétique, et
- déposer le polymère (12) sur la surface (1a) pour former un revêtement de surface (10), comprenant le polymère (12), sur la surface (1a) du ballonnet (1).

2. Procédé selon la revendication 1, dans lequel le polymère (12) est l'un des polymères suivants: un polyamide aliphatique ou aromatique, ou un polymère thermoplastique, de préférence un polymère thermoplastique modifié par un élastomère, et de préférence un polyétherblocamide.

3. Procédé selon l'une quelconque des revendications précédentes, dans lequel le polymère (12), dans la solution (13), présente une concentration comprise dans la plage de 1 % en poids à 30 % en poids.

4. Procédé selon l'une quelconque des revendications précédentes, dans lequel le polymère (12) de la solution (13) est déposé sur la surface (1a) par électrofilage.

5. Procédé selon l'une quelconque des revendications précédentes, dans lequel le ballonnet (1) comprend l'une des substances suivantes ou est formé de l'une des substances suivantes : un polyamide aliphatique ou aromatique ou un polymère thermoplastique modifié par un élastomère, tel qu'un polyétherblocamide.

6. Procédé selon l'une quelconque des revendications précédentes, dans lequel le revêtement de surface (10) est revêtu d'un médicament ou d'une composition contenant un médicament.

7. Procédé selon l'une quelconque des revendications précédentes, dans lequel le ballonnet (1) comprend au moins un conducteur électrique (30) sur la surface (1a) du ballonnet avant le dépôt du polymère (12), et, en particulier, le revêtement de surface (10) généré fait saillie au-delà d'une région (100) du revêtement de surface (10) recouvrant l'au moins un conducteur (30), selon une direction normale à la surface (1a) du ballonnet (1) dans une zone environnante (30a) de l'au moins un conducteur (30).

8. Ballonnet (1) pour un cathéter à ballonnet (2), le ballonnet (1) obtenu par le procédé selon l'une quelconque des revendications précédentes.

9. Cathéter à ballonnet (2), comprenant un ballonnet (1) selon la revendication 8 et un stent (3) disposé sur le revêtement de surface (10) du ballonnet (1).

10. Utilisation d'une solution (13), comprenant de l'acide trifluoroacétique en tant que solvant (11), et un polymère (12) dissous dans le solvant (11) pour revêtir une surface extérieure (1a) d'un ballonnet (1).
